# EUROPEAN PATENT APPLICATION

(11) **EP 4 574 842 A1**
(43) Date of publication of application: **25.06.2025**
(21) Application number: 23219972.9
(22) Date of filing: 22.12.2023
(51) Int. Cl.: C07K 16/28, A61P 13/12, A61K 47/68, C07K 16/44, A61K 39/00

(54) **BISPECIFIC CONJUGATE FOR ANTIGEN-SPECIFIC B CELL DEPLETION BY T CELLS**

(71) Applicant: Deutsches Rheuma-Forschungszentrum Berlin, 10117 Berlin (DE); Charité - Universitätsmedizin Berlin Körperschaft des öffentlichen Rechts, 10117 Berlin (DE)
(72) Inventor: CHENG, Qingyu, 10117 Berlin (DE); HIEPE, Falk, 10117 Berlin (DE); ALEXANDER, Tobias, 10117 Berlin (DE); RADBRUCH, Andreas, 10117 Berlin (DE); KHODADADI, Laleh, 10117 Berlin (DE)
(74) Representative: Hertin und Partner Rechts- und Patentanwälte PartG mbB

(57) **Abstract**

The invention relates to a bi-specific conjugate, comprising a binding agent that binds a T cell, and an antigen that binds an autoreactive B cell, wherein the antigen is a nucleic acid oligomer. The invention further relates to the use in the treatment and/or prevention of an autoimmune disease, preferably by selective depletion of anti-dsDNA specific B cells. The invention also relates to a pharmaceutical composition for use in the treatment and/or prevention of an autoimmune disease comprising the bispecific conjugate and a pharmaceutically acceptable carrier. The invention further relates to an in vitro method for killing an autoreactive B cell, the method comprising a mixture of an autoreactive B cell and a cytotoxic T cell with the bispecific conjugate or with a composition comprising said conjugate, wherein the antigen of said bispecific conjugate binds the autoreactive B cell and the binding agent of said bispecific conjugate binds and activates the cytotoxic T cell.

## Description

The invention lies in the field of molecular biology and medicine, more specifically in the field of immunobiology, infection medicine and the treatment of autoimmune diseases.

The invention relates to a bi-specific conjugate, comprising a binding agent that binds a T cell, and an antigen that binds an autoreactive B cell, wherein the antigen is a nucleic acid oligomer. The invention further relates to the bi-specific conjugate for use in the treatment and/or prevention of an autoimmune disease, preferably by selective depletion of anti-dsDNA specific B cells. The invention also relates to a pharmaceutical composition for use in the treatment and/or prevention of an autoimmune disease comprising the bispecific conjugate and a pharmaceutically acceptable carrier. The invention further relates to an in vitro method for killing an autoreactive B cell, the method comprising a mixture of an autoreactive B cell and a cytotoxic T cell with the bispecific conjugate or with a composition comprising said conjugate, wherein the antigen of said bispecific conjugate binds the autoreactive B cell and the binding agent of said bispecific conjugate binds and activates the cytotoxic T cell.

### BACKGROUND OF THE INVENTION

Autoimmune diseases result from aberrant immune system responses in which the body erroneously targets its healthy tissues, potentially resulting in severe health implications. To date, over 100 recognized autoimmune diseases exist, including prominent examples such as diabetes, multiple sclerosis, lupus, and rheumatoid arthritis, alongside rarer and diagnostically intricate conditions. The precise etiological underpinnings of these disorders remain elusive, likely arising from complex interactions between genetic predisposition and environmental influences. Specific demographic cohorts, such as women, individuals with familial autoimmune histories, and individuals exposed to specific environmental triggers, exhibit heightened susceptibility.

Management strategies are tailored to individual cases, with treatment objectives focused on symptom alleviation, immune system modulation, and preservation of the body's ability to combat diseases. Autoimmune disorders are prevalent and continue to be the subject of extensive research. Symptomatology is broad and can encompass manifestations such as fatigue, fever, joint pain, and skin rashes.

Given the heterogeneity of autoimmune diseases and their diverse symptomatic expressions, a comprehensive and multidisciplinary approach is imperative to effectively address these complex maladies and their ramifications on patients' lives.

Autoimmune diseases are intricately linked to the development and pathogenesis of both systemic and organ-specific autoimmune disorders, with B cells playing a pivotal role in these processes. While autoreactive B cells are commonly associated with autoantibody production, their primary pathogenetic contributions may lie in presenting autoantigens to T cells and secreting proinflammatory cytokines (Chan and Shlomchik, J Immunol., 1998). The activation of autoreactive B cells emerges as a critical and rate-limiting step in the pathogenesis of autoimmunity.

B cells play a critical role in promoting autoimmunity across a spectrum of diseases, from classical B cell-mediated autoimmune disorders like systemic lupus erythematosus (SLE) and rheumatoid arthritis (RA) to conditions previously not thought to involve B cells, such as diabetes and multiple sclerosis. Targeting B cells has emerged as a highly effective treatment strategy for autoimmune disorders in recent years, offering promise for disease course modification (Sfikakis PP et al., Curr Opin Rheumatol., 2005).

Self-tolerance mechanisms play a crucial role in shaping the B cell repertoire. B cell receptors (BCRs) are assembled through a stochastic process, resulting in receptors with unpredictable specificities, potentially including self-reactivity. Approximately 50% of BCRs may exhibit unacceptably high degrees of autoreactivity (Merrell K.T. et al., Immunity, 2006). Self-reactivity is managed through mechanisms such as clonal deletion, clonal anergy, and receptor editing. Anergy, a reduced ability to stimulate B cells via their antigen receptor, varies in degrees of self-reactivity.

B cell depletion therapies like rituximab have shown to be promising in treating autoimmune diseases by targeting B cells, which play crucial roles in inflammation and as precursors of plasmablasts and plasma cells that secrete autoantibodies. These therapies, however, can have varying outcomes and may not be effective in all cases, especially if long-lived plasma cells are refractory to therapies targeting B cells through depletion (e.g., monoclonal anti-CD20 antibody) or blockade of activation (e.g., monoclonal anti-BAFF/BLyS antibody) are responsible for the autoantibody production (Hiepe & Radbruch, Nat Rev Nephrol, 2016).

Therapies targeting B cells, such as anti-CD20 or anti-BAFF antibodies, affect both protective and autoreactive B cells unselectively. A solution can be the selective depletion of pathogenic B cells in an (auto)antigen-specific manner. This prevents the differentiation of pathogenic B cells into plasmablasts and plasma cells, which produce and secrete the corresponding pathogenic (auto)antibodies.

Chimeric autoantibody receptor (CAAR) T-cell therapies represent an alternative approach for the antigen-specific elimination of pathogenic plasma cells in the context of autoimmune diseases. For example, Ellebrecht et al. developed chimeric autoantibody receptors (CAARs) to engineer human T cells to target and eliminate autoreactive B lymphocytes in pemphigus vulgaris (PV). These CAAR-T cells demonstrated specific cytotoxicity against PV-related B cells *in vitro* and effectively eliminated them *in vivo.*

However, implementing CAAR-T cell technology has unveiled significant complexity and cost considerations. T cells derived from the patient have to be individually engineered ex *vivo* to express the autoantigen-based chimeric immunoreceptor, enabling targeted elimination of autoreactive B cells through B cell receptor-specific mechanisms. The complexity of CAAR T-cell therapy arises from the precise genetic engineering, the need for effective receptor design, and the requirement for fine-tuned immunomodulation to achieve a balanced and targeted autoimmune disease intervention. Consequently, alternative technologies and strategies are in development.

Autoimmune diseases characterized by immune responses against nuclear autoantigens comprise a complex interplay between the immune system and specific cellular components. High-titer autoantibodies have been pivotal in identifying the molecular targets involved in these diseases, revealing a remarkable specificity in the immune responses. Notably, these diseases are classified based on their impact on specific tissues or multiple tissues, revealing distinct patterns in autoantigen targeting. The kinetics of disease development indicate that immune responses against autoantigens often precede clinical symptoms, occurring in distinct phases that may involve a feedforward loop.

Furthermore, the presence of nucleic acids, particularly DNA and RNA, in immune complexes is a common feature across various rheumatic disease phenotypes. This includes systemic lupus erythematosus, where patient autoantibodies independently target multiple components of nucleoprotein complexes involved in mRNA splicing. The autoimmune response extends beyond individual components, often encompassing entire multimolecular complexes that play pivotal roles in cellular processes such as DNA replication, repair, and RNA processing.

Zocher et al. disclose a fusion protein, MOGxanti-CD3, that specifically targets autoreactive B cells by recognizing both the B cell receptors for the myelin oligodendrocyte glycoprotein (MOG) and CD3 on human T cells, leading to the redirection of lysis by T cells.

WO2002/16414 discloses a composition for the selective elimination of autoreactive B cells comprising at least one (poly)peptide construct consisting of at least two domains, wherein one of said domains comprises an autoreactive antigen or (a) fragment(s) thereof specifically recognized by the Ig receptors of said autoreactive B-cells and wherein one of said domains comprises an effector molecule capable of interacting with and/or of activating NK-cells, T-cells, macrophages, monocytes and/or granulocytes and/or capable of activating the complement system.

Despite these approaches, there still exists an unmet need for treating autoimmune diseases and developing approaches that selectively target and eliminate pathogenic B cells while preserving the essential protective functions of the immune system. Current strategies are diverse, given the complexity of autoimmune diseases, but they often lack specificity and can have varying effectiveness.

### SUMMARY OF THE INVENTION

In light of the prior art, one objective of the invention is to provide improved or alternative means for treating autoimmune diseases.

Another objective is the provision of means that improve the selective target and elimination of pathogenic B cells while sparing and preserving protective B cells.

Another problem underlying the invention is the provision of a standard treatment protocol applicable to a substantial patient population.

Another objective of the invention is the provision of means for treating and/or preventing autoimmune disease caused by and/or associated with nuclear and/or nucleic acid autoantigens.

These problems are solved by the features of the independent claims. Preferred embodiments of the present invention are provided by the dependent claims.

The invention, therefore, relates to a bispecific conjugate comprising a binding agent that binds a T cell and an antigen that binds an autoreactive B cell, wherein the antigen is a nucleic acid oligomer.

The present invention is based on the surprising finding that a bispecific conjugate, as described herein, can selectively kill autoreactive B cells by binding to a T cell, for example via binding CD4 or CD8 or fragments thereof, and simultaneously binding to an autoreactive B cell via an autoantigenic nucleic acid oligomer.

As demonstrated in the examples below, the inventors surprisingly found that the bispecific conjugate, consisting of a binding agent for T cells and an antigen-binding component for autoreactive B cells, could selectively deplete antigen-specific autoreactive B cells. While similar constructs existed before, the novel technical effect underlying the present invention lies in using the (auto)antigen comprising a nucleic acid oligomer component, conjugated to an antibody recognizing T cells, including, without limitation, anti-CD3, anti-CD4, anti-CD8, and others. This enables specific killing of B cells expressing a B cell receptor for the (auto)antigen, achieved through T cell-mediated cytotoxicity. This selective B cell depletion is antigen-specific, sparing other B cells, including protective ones.

In embodiments, the binding agent binds a component of the T cell receptor (TCR).

In embodiments, the binding agent of the conjugate binds a component of an αβ T cell receptor.

In embodiments, the binding agent of the conjugate binds a component of a γδ T cell receptor.

In embodiments, the binding agent binds and activates a cytotoxic T cell.

In embodiments, the binding agent binds to a cytotoxic T cell.

In embodiments, the binding agent binds to effector cytotoxic T cells, memory cytotoxic T cells, exhausted cytotoxic T cells, resident memory cytotoxic T cells, regulatory cytotoxic T cells, and/or tissue-resident cytotoxic T cells.

The bispecific ability of the conjugate to bind both a cytotoxic T cell and an autoreactive B cell represents a significant advantage, enabling a highly specific yet comprehensive target engagement.

In one aspect of the invention, the binding agent is or comprises an antibody or an antigen-binding fragment thereof.

In embodiments, the binding agent is or comprises an anti-CD3 antibody or an antigen binding fragment thereof.

The CD3 subunit complex is important in transducing antigen-recognition signals into the cytoplasm of T cells and in regulating the cell surface expression of the TCR complex. T cell activation through the antigen receptor (TCR) involves the cytoplasmic tails of the CD3 subunits CD3 gamma, CD3 delta, CD3 epsilon, and CD3 zeta. These CD3 subunits are structurally related members of the immunoglobulin superfamily encoded by closely linked genes on human chromosome 11. The CD3 components have long cytoplasmic tails that associate with cytoplasmic signal transduction molecules, and this association is mediated at least in part by a double tyrosine-based motif present in a single copy in the CD3 subunits. CD3 may play a role in TCR-induced growth arrest, cell survival, and proliferation. The CD3 antigen is present in 68-82% of normal peripheral blood lymphocytes, 65-85% of thymocytes, and Purkinje cells in the cerebellum. It is never expressed on B or NK cells.

In embodiments, the binding agent is or comprises an anti-CD4 or anti-CD8 antibody or an antigen-binding fragment thereof.

CD4 is primarily expressed in a subset of T-lymphocytes, also referred to as T helper cells, but may also be expressed by other cells in the immune system, such as monocytes, macrophages, and dendritic cells. However, CD4 may be used as a T cell marker, and used to bind T cells according to the present invention. At the tissue level, CD4 expression may be detected in the thymus, lymph nodes, tonsils, spleen, and specific regions of the brain, gut, and other nonlymphoid tissues. CD4 initiates or augments the early phase of T-cell activation through its association with the T-cell receptor complex and protein tyrosine kinase, Lck. It may also function as an important mediator of direct neuronal damage in infectious and immune-mediated diseases of the central nervous system.

CD8 is a type I transmembrane glycoprotein of the immunoglobulin family of receptors and plays an integral role in signal transduction and T cell differentiation and activation. CD8 is predominantly expressed on T cells as a disulfide-linked heterodimer of CD8alpha and CD8beta, where it functions as a co-receptor, along with T cell receptor (TCR), for major histocompatibility complex class I (MHC-I) molecules, whereas its counterpart, CD4, acts as a co-receptor for MHC-II molecules. CD8 may be used as a T cell marker, and used to bind T cells according to the present invention. CD8 exists on the cell surface, where the CD8-alpha chain is essential for binding to MHC-I. CD8 is also expressed on a subset of T cells, NK cells, monocytes, and dendritic cells as disulfide-linked homodimers of CD8alpha. Ligation of MHC-I/peptide complexes presented by antigen-presenting cells (APCs) triggers the recruitment of lymphocyte-specific protein tyrosine kinase (Lck), which leads to lymphokine production, motility, and cytotoxic T lymphocyte (CTL) activation. Once activated, CTLs play a crucial role in the clearance of pathogens and tumor cells.

The bispecific conjugate of the present invention preferably exhibits a notable advantage by demonstrating the capability to bind selectively to both T cells and a range of pathogenic B cells, directed against nucleic acid autoantigens. This characteristic facilitates targeted interactions with specific subsets of T cells, each possessing unique roles in immune responses. The versatility of the bispecific conjugate is underscored by its ability to engage subsets that are, e.g., involved in aiding other immune cells or directly eliminating harmful cells while maintaining precision in its functionality.

In embodiments, the antigen is bound by autoantibodies directed against the nucleic acid antigen, as may be found, by way of example, in patients with autoimmune diseases caused by and/or associated with a nucleic acid autoantigen.

In embodiments, the antigen is a dsDNA oligomer or a ssDNA oligomer, preferably a dsDNA oligomer.

Advantageously, the bispecific conjugate is effective in both forms, ssDNA or dsDNA, making it versatile and flexible for detecting different types of autoantibody targets.

In embodiments, the nucleic acid oligomer has a length of 20 to 300 nucleotides or base pairs.

In embodiments, the nucleic acid oligomer has a length of 20, 30, 40, 50, 60, 70, 80, 90, 100, 110, 120, 130, 140, 150, 160, 170, 180, 190, 200, 210, 220, 230, 240, 250, 260, 270, 280, 290, 300 nucleotides or base pairs. The length of the nucleic acid oligomer may also fall within a size range formed between any two endpoints mentioned in the list.

To the best of the inventors' knowledge, the present invention represents the first instance where the binding of a double-stranded (ds) nucleic acid oligomer with anti-DNA antibodies is length dependent.

In preferred embodiments of the invention, the nucleic acid oligomer has a length of 20 to 400 nucleotides or base pairs, more preferably 60 to 200 nucleotides or base pairs, most preferably 80 to 120 nucleotides or base pairs.

It was entirely surprising that these lengths of nucleic acid oligomers lead to more effective autoreactive B cell binding compared to oligos with a length of, for example, 9 bp. While a nucleic acid oligomer appears to be effective independent of the specific size or length of the nucleic acid, unexpectedly good results were obtained using oligomers as autoantigens with lengths of 50 to 200 bp, preferably 60 to 150 bp, more preferably 80 to 120 bp, or 80 to 100 bp.

Advantageously, the polynucleotide is not sequence-specific. Therefore, the binding of the oligonucleotide to the anti-dsDNA antibody is not sequence-dependent, thus offering a more flexible and versatile use of the bispecific conjugate.

In embodiments, the bi-specific conjugate is not sequence-specific.

In embodiments, the binding of the oligonucleotide to the anti-dsDNA antibody is not sequence-dependent.

In embodiments, the final concentration of the bispecific conjugate in the composition prior to administration is approx. 0.0016 µg/ml, 0.008 µg/ml, 0.04 µg/ml, 0.2 µg/ml, 1 µg/ml, 5 µg/ml, or 25 µg/ml. The concentration of the bispecific conjugate may also fall within a size range between any two endpoints mentioned in the list.

In embodiments, the final concentration of the nucleic acid oligomers ranges between 0.49 nM and 500 nM. In embodiments, the final concentration of the nucleic acid oligomers is 0.49 nM, 1 nM, 2 nM, 3 nM, 4 nM, 5 nM, 10 nM, 20 nM, 30 nM, 40 nM, 50 nM, 100 nM, 150 nM, 200 nM, 250 nM, 300 nM, 350 nM, 400 nM, 450 nM, or 500 nM. The final concentration of the nucleic acid oligomer may also fall within a size range formed between any two endpoints mentioned in the list.

In preferred embodiments, the final concentration of the nucleic acid oligomers is higher than 20 nM.

In embodiments, the bi-specific agent of the invention leads to a selective depletion of autoreactive B cells. In embodiments, the selective depletion of autoreactive B cells can be analyzed by flow cytometry, e.g., FACS.

In embodiments, the antigen of the bi-specific agent of the invention is a dsDNA oligomer which is bound by a pathogenic autoantibody. In some embodiments, the binding between anti-dsDNA antibodies and nucleic acid oligomers may be detected by Enzyme-Linked Immunosorbent Assay (ELISA); see examples below.

Therefore, a skilled person is capable of determining whether any given embodiment of the invention fulfills the functional characteristics of the bi-specific agent as claimed, for example the selective depletion of autoreactive B cells and/or whether any given dsDNA oligomer is bound by a pathogenic autoantibody.

In embodiments, the antigen is associated with an autoimmune disease.

In preferred embodiments, the autoimmune disease is a systemic autoimmune disease comprising anti- nucleic acid autoantibodies.

In preferred embodiments, the systemic autoimmune disease comprising anti-nucleic acid autoantibodies is systemic lupus erythematosus (SLE).

These medical conditions have been shown to be associated with autoantibodies directed against nuclear, preferably nucleic acid, autoantigens. Some common nucleic acid autoantigens associated with autoimmune diseases include dsDNA, ssDNA, RNA, histones, nucleosomes, snRNPs, and ribosomal RNA. Notably, the presence of autoantibodies against these nucleic acid antigens is often used as a diagnostic marker for specific autoimmune diseases, aiding identifying and classifying these conditions.

In embodiments, the bispecific conjugate is used in the treatment and/or prevention of an autoimmune disease. In preferred embodiments, the autoimmune disease is a systemic autoimmune disease. In preferred embodiments, the systemic autoimmune disease is a systemic autoimmune disease comprising anti- nucleic acid autoantibodies. In preferred embodiments, the autoimmune disease is systemic lupus erythematosus (SLE).

A systemic autoimmune disease is characterized by aberrant immune responses wherein the immune system erroneously recognizes and targets the body's tissues. Unlike organ-specific autoimmune disorders, systemic autoimmune diseases manifest as a pervasive assault on multiple organs and physiological systems. The pathophysiology involves systemic inflammation, giving rise to a spectrum of symptoms and complications that impact joints, skin, kidneys, blood vessels, and various organs.

Systemic lupus erythematosus (SLE), commonly known as lupus, is an autoimmune disease characterized by the immune system mistakenly attacking healthy tissues in various body parts. Symptoms, ranging from mild to severe, include joint pain, fever, chest pain, hair loss, fatigue, and a characteristic red rash, often on the face. Fluctuating between periods of illness (flares) and remission is common. The cause of SLE is unclear but is believed to involve a combination of genetic and environmental factors, with female sex hormones, sunlight, smoking, vitamin D deficiency, and certain infections playing a role. Diagnosis is challenging and relies on a combination of symptoms and laboratory tests. Treatment aims at managing symptoms and may involve NSAIDs, corticosteroids, immunosuppressants, hydroxychloroquine, and methotrexate. While there is no cure, experimental treatments exist. SLE is known as "the great imitator" due to its varied and unpredictable symptoms, making diagnosis elusive. It affects women more frequently, with distinct symptom variations between genders. The disease's prevalence varies globally, with higher rates among certain ethnic groups. Lupus significantly increases the risk of cardiovascular disease, and while it can impact pregnancies, successful outcomes are possible.

Patients with SLE have been shown to exhibit autoantibodies targeting nuclear nucleic acid antigens, including DNA-containing nucleosomes and RNA-containing entities such as small nuclear ribonucleoproteins (snRNPs), cytoplasmic RNPs (SS-A/Ro), and ribosomes. Antibodies also target negatively charged phospholipids. Furthermore, anti-double-stranded DNA (anti-dsDNA) antibodies hold significant relevance in systemic lupus erythematosus (SLE), with approximately 70-80% of the patients being positive for these antibodies, and titers commonly correlating with disease activity (Mustelin T et al., Front Immunol., 2019). A noteworthy characteristic of lupus autoantigens is their absence of strict subcellular localization, as they may be found exclusively in the nucleus, cytosol, or associated with the membrane, with distribution between the nucleus and cytosol varying under different conditions.

Arthritis is a broad term encompassing various joint disorders characterized by symptoms such as joint pain, stiffness, redness, warmth, swelling, and reduced range of motion. Onset can be gradual or sudden, and there are over 100 types of arthritis. The most prevalent forms include osteoarthritis, associated with age and affecting joints like fingers, knees, and hips, and rheumatoid arthritis, an autoimmune disorder commonly affecting hands and feet. Other types include gout, lupus, fibromyalgia, and septic arthritis, all falling under the category of rheumatic diseases. Treatment approaches involve joint rest, alternating between applying ice and heat, weight loss, and exercise. Medications, such as ibuprofen and paracetamol, may be recommended based on the specific type of arthritis. In some cases, joint replacement surgery may be beneficial. Several reports have demonstrated that memory B cells making anti-DNA antibodies and anticitrullinated protein antibodies enhance arthritis (Kuhn K et al., J Clin Invest, 2006).

Several methods are employed in managing autoimmune diseases, including immunosuppressive agents, short-term anti-inflammatory interventions, plasmapheresis, stem cell transplants, and lifestyle adjustments. CAR-T cell therapy has shown promising results in treating certain cancers, and the development of CARs for treating autoimmune diseases is evolving (G. Schett et al., The Lancet, 2023). However, most medications used to treat autoimmune diseases act through immune system suppression. Although effective in tempering autoimmune responses, this approach heightens susceptibility to infections and potential immunosuppressive-related complications. Additionally, pharmaceutical usage is accompanied by diverse side effects, including gastrointestinal disturbances, cutaneous manifestations, weight fluctuations, and mood alterations. Furthermore, limitations in efficacy are evident in certain therapeutic approaches. Prolonged or lifelong medication requirements raise concerns regarding medication dependence and long-term side effects.

Hence, there is a compelling need to develop innovative approaches for the treatment of these conditions.

The present invention, therefore, suggests the possibility of establishing a standardized treatment protocol for all patients without the need for gene modification of T cells in humans. This method can be extended beyond (auto)antigen-specific B cell depletion to selectively target other pathogenic cells based on the expression of characteristic molecules on their surface. This broader application enhances the potential versatility and effectiveness of the proposed therapeutic approach.

Covalent linkers play a crucial role in the bioconjugation of oligonucleotides to binding agents, facilitating the formation of stable and specific connections between the two molecular entities.

In embodiments, the binding agent and the antigen are associated by a covalent linker. Covalent linkages are typically more stable than non-covalent interactions, ensuring the conjugate remains intact under various physiological conditions. Moreover, the covalent linker was precisely engineered to form strong, specific bonds, minimizing off-target effects and enhancing the selectivity of the conjugate.

In embodiments, common types of covalent linkers may be used for bioconjugation, such as cleavable linkers, e.g., disulfide linkers or photocleavable linkers; spacer linkers, e.g., glycan linkers, aliphatic spacers, polyethylene glycol, or peptide linkers; click chemistry linkers, e.g., azide and alkyne linkers; amide linkers; thiol-reactive linkers; and/or biotin-streptavidin linkers.

In embodiments, the binding agent and the antigen are associated by a glycan linker.

In embodiments, the glycan linker can be conjugated by the Thiol-monoclonal-antibody (Thiol-mAb) technology. In embodiments, the conjugation occurs via a thiol-reactive group, often a maleimide, which forms a stable covalent bond with the thiol group on the antibody. The advantage of thiol-mab technology lies in its ability to achieve site-specific conjugation, minimizing the risk of interfering with the antibody's binding regions or affecting its biological activity. This precision is beneficial for applications like antibody-drug conjugates (ADCs), where the payload needs to be attached in a controlled and reproducible manner.

In one aspect, the invention relates to a pharmaceutical composition for use in the treatment and/or prevention of an autoimmune disease, comprising the bispecific conjugate according to the invention and a pharmaceutically acceptable carrier.

In embodiments, the invention provides a method for prevention and/or treatment of a medical condition caused or exacerbated by an autoreactive B cell, the method comprising delivery to an individual a bispecific agent as disclosed herein.

In another aspect, the invention relates to an *in vitro* method for killing an autoreactive B cell, comprising contacting a mixture of an autoreactive B cell and a cytotoxic T cell with the bispecific conjugate or with a composition comprising said conjugate, wherein the antigen of said bispecific conjugate binds the autoreactive B cell and the binding agent of said bispecific conjugate binds and activates the cytotoxic T cell.

Preferably, the bispecific agent of the invention is not immunogenic in humans.

The person skilled in the art is familiar with methods for the generation of antibodies and fragments thereof, which are specific to a given target. Antibodies are readily created in animals such as rabbits or mice by immunization with the gene product or a fragment thereof. Immunized mice can provide sources of B cells to manufacture hybridomas, which in turn are cultured to produce large quantities of monoclonal antibodies. These antibodies and the nucleic acids encoding them can be used to generate the bispecific agent according to the present invention. For example, in embodiments, a nucleic acid encoding an antibody or part thereof, which forms the binding agent, can be cloned and then ligated to a nucleic acid encoding an antigen, followed by an expression in an appropriate expression system to generate the bispecific conjugate of the present invention. In preferred embodiments, the antibody or fragment thereof used in the bispecific agent is a recombinant antibody or fragment thereof.

The invention further relates to the use of a bispecific agent according to the invention in the elimination *in vivo* of specific antibody-producing cells, wherein said antibod(y)ies target at least a T cell, an autoreactive B cell, or a combination thereof, even where such cells and the antibodies they produce are not associated with a medical condition, such as an autoimmune disease. For example, bispecific agents, according to the invention, may be used in animal models, for example, in situations in which it is desired to remove or reduce plasma cells and the antibodies they produce for reasons unrelated to a medical condition, but which are advantageous for the use of the animal model.

Embodiments and features of the invention described concerning the bispecific agent, the *in vivo* methods, and kits are considered to be disclosed with respect to every other aspect of the disclosure, such that features characterizing the bispecific agent, may be employed to characterize the methods or kit and vice-versa. The various aspects of the invention are unified by, benefit from, are based on and/or are linked by the common and surprising finding of the depletion of autoreactive B cells and long-lived autoreactive plasma cells through the delivery of a bispecific agent comprising a binding agent that binds a T cell, and an antigen that binds an autoreactive B cell, wherein the antigen is a nucleic acid oligomer.

### DETAILED DESCRIPTION OF THE INVENTION

All cited documents of the patent and non-patent literature are hereby incorporated by reference in their entirety.

The invention is directed to a bispecific conjugate, comprising a binding agent that binds a T cell and an antigen that binds an autoreactive B cell, wherein the antigen is a nucleic acid oligomer.

The invention further relates to the use of said a bispecific conjugate in the treatment and/or prevention of an autoimmune disease.

Herein, a "bispecific conjugate" or "bispecific agent" in the context of the present invention refers to a molecule or a complex designed to recognize and bind to two different targets, e.g., two different cells. The bispecific conjugate consists of two specific components to create a single molecule or complex. In embodiments, the bispecific conjugate comprises a binding agent and an antigen. The person skilled in the art is aware of different techniques to link two components, e.g., a binding agent and an antigen, to obtain a bispecific conjugate.

An "antigen (Ag)" refers to a compound, composition, or substance that is bound by an antibody. An antigen stimulating an "immune response" or "immune reaction," such as the production of antibodies or a T cell response in an animal or human subject, is also referred to as an "immunogen." Antigens and/or immunogens usually comprise protein, lipid and/or carbohydrate structures, however, nucleic acids may also represent autoantigens. An antigen usually has several antigenic substructures, the region of an antigen to which a binding agent binds and which are referred to as "determinants" or "epitopes". Thereby, epitopes can be formed both from contiguous structures, such as an amino acid sequence, or noncontiguous structures, such as two or more amino acid sequences juxtaposed by the tertiary folding of a protein. Therefore, epitopes can be composed of two distinct proteins in proximity or a protein and another immunogen as a complex.

Herein, "nucleic acid" may preferably refer to DNA (deoxyribonucleic acid), gDNA (genomic deoxyribonucleic acid), RNA (ribonucleic acid), gRNA (genomic ribonucleic acid), mRNA (messenger ribonucleic acid) and cDNA (complementary deoxyribonucleic acid synthesized from RNA template), or any combination thereof. Nucleic acid sequences refer herein to a consecutive array of nucleotides, wherein the nucleotides are distinguished by their nucleobases into guanine (G), adenine (A), cytosine (c) and thymine (T) in DNA and uracil (U) -instead of thymine- in RNA. A nucleic acid sequence may herein also refer to the sequence of consecutive letters (comprised of G, A, C, and T or U) that represent the actual sequence of consecutive nucleic acids in a strand of DNA or RNA. This nucleic acid sequence may be biochemically and bioinformatically identified and characterized using DNA or RNA sequencing. The sequencing analysis may also involve the comparison of the obtained nucleic acid sequence and one or more reference nucleic acid sequences. As used herein, "nucleic acid" shall mean any nucleic acid molecule, including, without limitation, DNA, RNA, and hybrids or modified variants thereof.

The term "nucleic acid oligomer" or "oligonucleotide" refers to an oligomer or polymer of nucleic acids with its ordinary meaning in the art, including without limitation ribonucleic acid (RNA) or deoxyribonucleic acid (DNA) or any mimetic or structurally modified nucleic acid thereof, including, without limitation DNA, messenger RNA (mRNA), RNA, genomic RNA (gRNA), transfer RNA (tRNA), ribosomal RNA (rRNA), microRNA (miRNA), small interfering RNA (siRNA), single guide RNA (sgRNA), piwi-interacting RNA (piRNA), small nuclear RNA (snRNA), plus-strand RNA (RNA(+)), minus-strand RNA (RNA(-)), genomic DNA (gDNA), complementary DNA (cDNA), recombinant polynucleotides, branched polynucleotides, plasmids, nucleic acid probes and primers. The term includes, without limitation, single and double-stranded (ssDNA, dsDNA) nucleic acid oligomers.

To ensure the stability of the oligonucleotides and to prevent degradation, the oligonucleotides can be substituted with different modifications into native phosphodiester oligodeoxyribonucleotide and ribonucleotide polymers. These modifications may be incorporated during oligonucleotide synthesis. Non-limiting examples of modifications include phosphorothioate (PS) bonds, 2'-O-Methyl (2'OMe), 2' fluoro bases, inverted dT and ddT, phosphorylation, and C3 spacer.

A "T cell," also termed "T lymphocyte," is an immune cell belonging to the group of lymphocytes. A T cell can be a thymocyte, immature T lymphocyte, mature T lymphocyte, resting T lymphocyte, cytokine-induced killer cell (CIK cell), or activated T lymphocyte. T cells originate from the bone marrow and migrate via the bloodstream to the thymus, where they generate T cell receptors (TCR) and undergo a positive and negative selection in which the cells that show high affinity to endogenous proteins are degraded. T cells may be a T helper (Th; CD4+ T cell) cell, for example, a T helper (Th) cell, such as a TH1, TH2, TH3, TH17, TH9, or TFH cell. The T cell can be a cytotoxic T cell (CTL; CD8+ T cell), CD4+CD8+ T cell, CD4 CD8 T cell, or a regulatory T cell (reg) or any other subset of T cells such as a cytokine-induced killer (CIK) cell which is typically a CD3- and CD56-positive, non-major histocompatibility complex (MHC)- restricted, natural killer (NK)-like T lymphocyte. The T cell can be a naive, effector, memory, effector storage, central storage, and memory stem T cell.

A "T cell receptor" (TCR) is a protein complex found on the surface of T cells that is responsible for recognizing and binding fragments of antigens bound to major histocompatibility complex (MHC) molecules, e.g., on the surface of APCs. The TCR is a heterodimer, which comprises a TCR alpha and TCR beta protein chain or a TCR gamma and a TCR delta protein chain. T cells with a TCR composed of a TCR alpha and a TCR beta protein chain account for approx. 95% of the T cell population in the peripheral blood. The heterodimer subunits consist of a constant domain (C) and a variable domain (V), a transmembrane domain, and a short C-terminal cytoplasmic region. The N-terminal ends of the chains belonging to the C domain penetrate the cell membrane into the cytoplasmic space and anchor the receptor. A disulfide bridge in the constant region extracellularly links the two subunits.

"B cells" or "B lymphocytes" constitute a subset of white blood cells within the lymphocyte category, operating in the humoral immunity sector of the adaptive immune system. These cells play a crucial role by producing antibody molecules that can be secreted or embedded in the plasma membrane, serving as B-cell receptors. Upon activation by an antigen, B cells proliferate and transform into antibody-secreting effector cells called plasmablasts or plasma cells. B cells act as professional antigen-presenting cells, presenting antigens and secreting cytokines. In mammals, B cells mature in the bone marrow. Unlike T cells and natural killer cells, B cells express B cell receptors (BCRs) on their membrane, facilitating the binding to foreign antigens and initiating an antibody response. Development starts from hematopoietic stem cells in the bone marrow, progressing through various stages with specific gene expressions and rearrangements. During development, B cells undergo positive and negative selections to ensure proper maturation, involving BCRs in both processes. Positive selection relies on antigenindependent signaling through pre-BCR and BCR, while negative selection involves the binding of self-antigens to BCRs, leading to various outcomes such as clonal deletion, receptor editing, anergy, or ignorance. After development, immature B cells migrate to the spleen as transitional B cells, transitioning from T1 to T2. These cells further differentiate into follicular (FO) B cells or marginal zone (MZ) B cells based on signals received through BCRs and other receptors. B cell activation occurs in secondary lymphoid organs like the spleen and lymph nodes. T celldependent activation involves the interaction of B cells with T cells, resulting in a prolonged but high-affinity antibody response. T cell-independent activation, on the other hand, is faster but produces antibodies with lower affinity. Activated B cells undergo differentiation into plasmablasts during the extrafollicular response or form germinal centers for extensive proliferation, immunoglobulin class switching, and affinity maturation, generating long-lived plasma cells and memory B cells. B cell types include plasmablasts, plasma cells, memory B cells, B-2 cells (FO and MZ B cells), B-1 cells, and regulatory B (Breg) cells, each with specific functions and locations within the body. B cell-related pathologies include autoimmune diseases resulting from abnormal B cell recognition of self-antigens and malignant transformations leading to cancers such as chronic lymphocytic leukemia, lymphomas, and multiple myeloma. Abnormal B cells may also cause conditions like diffuse large B-cell lymphomas.

An "autoreactive B cell" refers to a specialized white blood cell belonging to the B lymphocyte subtype, which exhibits an aberrant immunological response by recognizing and reacting against self-antigens-endogenous molecules or structures present within the body's own tissues. This deviance from normal immune tolerance mechanisms can lead to the activation of autoreactive B cells, resulting in an immune response directed against the body's own cells and tissues. The dysregulation of autoreactive B cells is implicated in the pathogenesis of autoimmune diseases, where the immune system erroneously targets and damages host tissues, contributing to chronic inflammation and associated pathological manifestations.

As used herein, an "antibody" generally refers to a protein consisting of one or more polypeptides substantially encoded by immunoglobulin genes or fragments of immunoglobulin genes. Where the term "antibody" is used, the term "antibody fragment" may also be considered to be referred to. The recognized immunoglobulin genes include the kappa, lambda, alpha, gamma, delta, epsilon, and mu constant region genes and the myriad immunoglobulin variable region genes. Light chains are classified as either kappa or lambda. Heavy chains are classified as gamma, mu, alpha, delta, or epsilon, defining the immunoglobulin classes IgG, IgM, IgA, IgD, and IgE. The basic immunoglobulin (antibody) structural unit is known to comprise a tetramer or dimer. Each tetramer is composed of two identical pairs of polypeptide chains, each pair having one "light" (L) (about 25 kD) and one "heavy" (H) chain (about 50-70 kD). The N-terminus of each chain defines a variable region of about 100 to 1 10 or more amino acids, primarily responsible for antigen recognition. The terms "variable light chain" and "variable heavy chain" refer to these variable regions of the light and heavy chains, respectively. A variable region of an antibody refers to the variable region of the antibody light chain or the variable region of the antibody heavy chain, alone or in combination. The variable regions of the heavy and light chains each consist of four framework regions (FR) connected by three complementarity determining regions (CDRs), also known as hypervariable regions. The CDRs in each chain are held together in close proximity by the FRs and, with the CDRs from the other chain, contribute to the formation of the antigen-binding site of antibodies. Optionally, the antibody, or the immunological portion of the antibody, can be chemically conjugated to or expressed as a fusion protein with other proteins.

The term "autoantibody" refers to proteins composed of one or more polypeptides encoded by immunoglobulin genes or their fragments, recognizing self-antigens. In contrast to conventional antibodies, which typically target foreign substances, autoantibodies deviate by exhibiting a marked affinity for endogenous molecules, contributing to the pathogenesis of autoimmune diseases. Autoantibodies originate from a breakdown in immune tolerance mechanisms, where B cells recognizing self-antigens evade elimination and undergo clonal expansion. Having escaped central and peripheral tolerance checkpoints, these self-reactive B cells play a pivotal role in autoimmune diseases. Notably, defects in post-germinal center tolerance mechanisms are implicated in generating highly somatically mutated and class-switched autoantibodies. The effector functions of autoantibodies, akin to antibodies, are contingent on their isotype. While IgA and IgE autoantibodies are associated with specific autoimmune diseases, IgM and IgG autoantibodies prevail more broadly. In situ immune complexes, arising from the interaction of autoantibodies with self-antigens within tissues, can activate complement pathways and Fc receptors, leading to inflammation and damage. Importantly, through their distinct reactivity to self-antigens, autoantibodies underscore the aberrant immune responses characterizing autoimmune conditions, distinguishing them from the typical immune responses exhibited by conventional antibodies targeting foreign invaders. Commonly autoantibody-associated diseases may include systemic lupus erythematosus, Sjörgen's syndrome, CREST syndrome, myopathy, sclerosis, vasculitis, arthritis, polymyositis, dermatomyositis, and scleroderma.

"Autoimmune disorders" or "autoimmune diseases" manifest when the immune system erroneously targets and assaults healthy bodily components, with the identification of over 80 distinct types. They can affect various organs, leading to symptoms like fatigue, fever, joint pain, and skin rashes. Diagnosing involves medical history, physical exams, lab tests, and imaging. Causes are multifactorial, involving genetics, environment, hormones, infections, and immune dysregulation. Treatment aims to manage symptoms and control the immune response, but no cure exists. Prevalence is estimated at 10%, with women more affected. Common autoimmune diseases include but are not limited to celiac disease, type 1 diabetes, Graves' disease, inflammatory bowel diseases, multiple sclerosis, rheumatoid arthritis, and lupus. The complex etiology involves genetic predisposition, environmental factors, hormonal influences, infections, and dysregulated immune responses. Diagnosing autoimmune diseases is challenging due to diverse symptoms, and a multidimensional approach is often needed for management.

These conditions exhibit a propensity to impact diverse organs, resulting in manifestations such as fatigue, pyrexia, arthralgia, and dermal eruptions. The diagnostic process is intricate, encompassing meticulous scrutiny of medical history, physical examinations, laboratory analyses, and imaging modalities. Etiological factors are intricate and multifaceted, encompassing genetic predisposition, environmental influences, hormonal dynamics, infectious agents, and dysregulation of the immune milieu. Therapeutic interventions primarily strive to alleviate symptoms and modulate the immune response, notwithstanding a definitive cure. Prominent instances of autoimmune maladies comprise celiac disease, type 1 diabetes, Graves' disease, inflammatory bowel diseases, multiple sclerosis, rheumatoid arthritis, and lupus. The intricate etiological panorama involves genetic predisposition, environmental determinants, hormonal perturbations, infectious agents, and aberrations in immune homeostasis. The intricate symptomatology and heterogeneity of autoimmune diseases render their diagnosis a formidable task, necessitating a comprehensive and multidisciplinary approach for effective management.

In embodiments, the bispecific conjugate is used to treat and/or prevent autoimmune diseases. In embodiments, these autoimmune diseases are systemic lupus erythematosus (SLE), arthritis, polymyositis, dermatomyositis, Sjogren's syndrome, scleroderma, and systemic vasculitis.

A "covalent linker" refers herein to the linkage of two molecules (e.g., a binding agent and an antigen) through a covalent bond. A covalent bond is a chemical bond in which electrons are exchanged to form electron pairs between atoms; in chemistry, it refers to the interatomic bond formed by sharing a pair of electrons between two atoms. This bond is formed by the electrostatic attraction of their nuclei to the same electrons. A covalent bond occurs when the bonded atoms have a lower total energy than widely separated atoms.

As used herein, "treatment" or "treating" includes any beneficial or desirable effect on the symptoms or pathology of a disease or pathological condition and may include even minimal reductions in one or more measurable markers of the disease or condition being treated. Treatment can optionally involve either the reduction or amelioration of symptoms of the disease or condition or the delaying of the progression of the disease or condition. "Treatment" does not necessarily indicate complete eradication or cure of the disease, condition, or associated symptoms.

As used herein, "prevent" and similar words such as "prevented," "preventing," or "prophylactic," etc., indicate an approach for preventing, inhibiting, or reducing the likelihood of the occurrence or recurrence of a disease or condition. It also refers to delaying the onset or recurrence of a disease or condition or delaying the occurrence or recurrence of the symptoms of a disease or condition. As used herein, "prevention" and similar words also include reducing the intensity, effect, symptoms and/or burden of a disease or condition prior to onset or recurrence of the disease or condition.

A "pharmaceutical composition" refers to a composition formulated in pharmaceutically acceptable or physiologically acceptable solutions for administration to a cell or an animal, either alone or in combination with one or more other therapy modalities. It will also be understood that, if desired, the compositions of the invention may be administered in combination with other agents as well, such as cytokines, growth factors, hormones, small molecules, chemotherapeutics, pro-drugs, drugs, antibodies, or other various pharmaceutically active agents. There is virtually no limit to other components that may also be included in the compositions, provided that the additional agents do not adversely affect the ability of the composition to deliver the intended therapy.

The bispecific conjugate according to the invention may also be administered in a pharmaceutical composition prepared for administration to a subject and which comprise a therapeutically effective amount of one or more of the compounds disclosed herein. In embodiments, the pharmaceutical compositions are useful for treating or preventing an autoimmune disease related to the presence of autoreactive B cells. The therapeutically effective amount of the bispecific conjugate depends on the route of administration, the species of the subject, and the physical characteristics of the subject being treated. Specific factors that can be considered include weight, diet, and concurrent medications of a subject. The relationship of these factors to determining a therapeutically effective amount of a bispecific conjugate is understood by those skilled in the art.

The compositions contemplated herein may comprise one or more polypeptides, polynucleotides, vectors comprising the same genetically modified immune effector cells, etc., as contemplated herein. Compositions include, but are not limited to, pharmaceutical compositions.

Pharmaceutical compositions comprising a bispecific conjugate for administration to a subject can include, in embodiments, at least one further pharmaceutically acceptable additive such as carriers, thickeners, diluents, buffers, preservatives, surface active agents, and the like in addition to the molecule of choice. Pharmaceutical compositions can also include one or more additional active ingredients such as antimicrobial agents, anti-inflammatory agents, anesthetics, and the like. The pharmaceutically acceptable carriers useful for these formulations are conventional. The person skilled in the art is aware of compositions and formulations suitable for pharmaceutical delivery of the bi-specific agent disclosed herein. In general, the nature of the carrier will depend on the particular mode of administration being employed. For instance, parenteral formulations usually contain injectable fluids that include pharmaceutically and physiologically acceptable fluids such as water, physiological saline, balanced salt solutions, aqueous dextrose, glycerol, or the like as a vehicle. For solid compositions (for example, powder, pill, tablet, or capsule forms), conventional non-toxic solid carriers can include, for example, pharmaceutical grades of mannitol, lactose, starch, or magnesium stearate. In addition to biologically neutral carriers, pharmaceutical compositions to be administered can contain minor amounts of non-toxic auxiliary substances, such as wetting or emulsifying agents, preservatives, and pH buffering agents and the like, for example, sodium acetate or sorbitan monolaurate.

The bispecific conjugate can, in embodiments, be combined with pharmaceutically acceptable carrier substances as required to approximate physiological conditions, such as pH adjusting and buffering agents, tonicity adjusting agents, wetting agents, and the like, for example, sodium acetate, sodium lactate, sodium chloride, potassium chloride, calcium chloride, sorbitan monolaurate, and triethanolamine oleate. For solid compositions comprising the bi-specific agent, conventional non-toxic pharmaceutically acceptable vehicles can be used, which include, for example, pharmaceutical grades of mannitol, lactose, starch, magnesium stearate, sodium saccharin, talcum, cellulose, glucose, sucrose, magnesium carbonate, and the like. Liquid pharmaceutical compositions, whether they are solutions, suspensions, or other like forms, may include one or more of the following: sterile diluents such as water for injection, saline solution, preferably physiological saline, Ringer's solution, isotonic sodium chloride, fixed oils such as synthetic mono or diglycerides which may serve as the solvent or suspending medium, polyethylene glycols, glycerin, propylene glycol or other solvents; antibacterial agents such as benzyl alcohol or methylparaben; antioxidants such as ascorbic acid or sodium bisulfite; chelating agents such as ethylenediaminetetraacetic acid; buffers such as acetates, citrates or phosphates and agents for the adjustment of tonicity such as sodium chloride or dextrose. The parenteral preparation can be enclosed in ampoules, disposable syringes, or multiple dose vials made of glass or plastic. An injectable pharmaceutical composition is preferably sterile.

A bispecific conjugate or a bispecific conjugate comprised in a (pharmaceutical) composition can be administered to subjects by a variety of mucosal administration modes, including by oral, rectal, intraocular, intranasal, intrapulmonary, or transdermal delivery, intramuscular, intraocular, subcutaneous, intravenous, intra-arterial, intra-articular, intraperitoneal, intrathecal, intracerebroventricular, or parenteral routes.

In accordance with the disclosure herein, a prophylactically or therapeutically effective amount of the bispecific agent may, in embodiments, be administered to a subject in need of such treatment for a time and under conditions sufficient to prevent, inhibit, and/or ameliorate a selected condition or one or more symptom(s) thereof, wherein the condition is an autoimmune disease.

The attending clinician can vary dosage to maintain a desired concentration at a target site (for example, the lungs or systemic circulation). Higher or lower concentrations can be selected based on the mode of delivery, for example, trans-epidermal, rectal, oral, pulmonary, or intranasal delivery versus intravenous or subcutaneous delivery. Dosage can also be adjusted based on the release rate of the administered formulation, for example, of an intrapulmonary spray versus powder, sustained release oral versus injected particulate or transdermal delivery formulations, and so forth.

The instant disclosure also includes kits, packages, and multi-container units containing the herein-described bispecific conjugate or pharmaceutical compositions comprising the same and/or means for administering the same for use in the prevention and treatment of conditions described herein and other conditions in human subjects.

It will be understood that particular embodiments described herein are shown by way of illustration and not as limitations of the invention. The principal features of this invention can be employed in various embodiments without departing from the scope of the invention. Those skilled in the art will recognize or be able to ascertain, using most routine study, numerous equivalents to the specific procedures described herein. Such equivalents are considered to be within the scope of this invention and are covered by the claims. All publications and patent applications mentioned in the specification indicate the skill level of those skilled in the art to which this invention pertains. All publications and patent applications are herein incorporated by reference to the same extent as if each individual publication or patent application was specifically and individually indicated to be incorporated by reference.

The use of the word "a" or "an" when used in conjunction with the term "comprising" in the claims and/or the specification may mean "one," but it is also consistent with the meaning of "one or more," "at least one," and "one or more than one." The use of the term "or" in the claims is used to mean "and/or" unless explicitly indicated to refer to alternatives only or the alternatives are mutually exclusive. However, the disclosure supports a definition of only alternatives and "and/or." Throughout this application, where relevant, the term "about" indicates that a value includes the inherent variation of error for the device, the method employed to determine the value or the variation among the study subjects.

### FIGURES AND EXAMPLES

The invention is further described by the following figures and examples. These are not intended to limit the scope of the invention but represent preferred embodiments of aspects of the invention provided for a greater illustration of the invention described herein.

### Brief description of the Figures:

**Figure 1****:** Binding affinity of anti-dsDNA antibodies with distinct lengths of nucleic acid oligomers.
**Figure 2****:** Selective depletion of anti-dsDNA-specific B cells ex *vivo.*
**Figure 3****:** Selective depletion of anti-dsDNA-specific B cells *in vivo.*
**Figure 4****:** Long-term clinical effect of selective depletion of anti-dsDNA-specific B cells *in vivo.*

### Detailed description of the Figures:

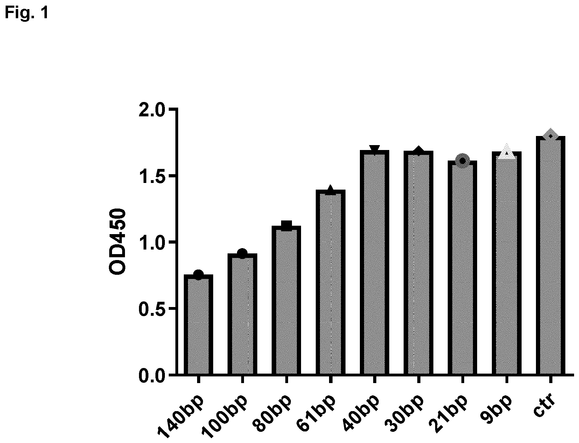

**Figure 1****:** Binding affinity of anti-dsDNA antibodies with distinct lengths of nucleic acid oligomers. Anti-dsDNA positive SLE patient sera were incubated with different lengths of nucleic acid oligomer with the concentration of 200 nM and then detected by Elisa. 'ctr' was the sample without nucleic acid oligomer incubation.

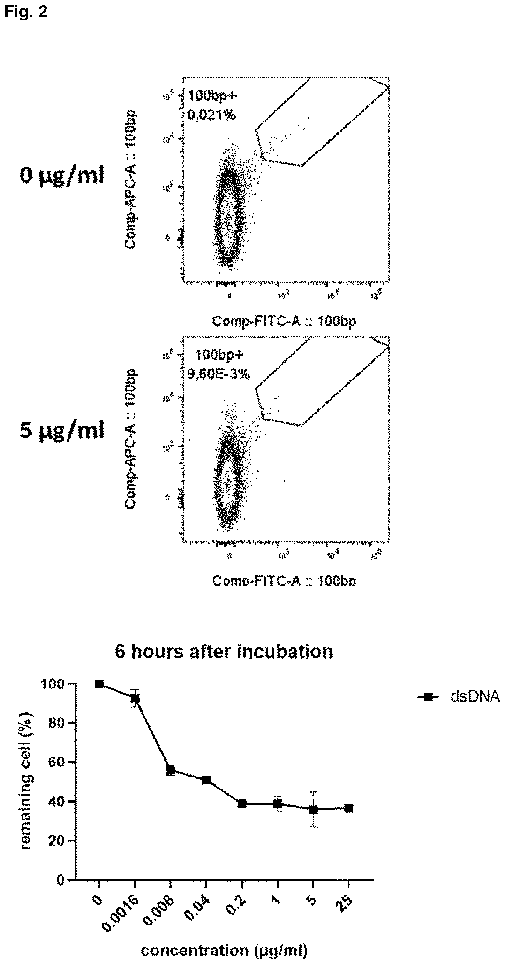

**Figure 2****:** Selective depletion of anti-dsDNA-specific B cells ex *vivo.* Mouse splenocytes were isolated from lupus-prone NZB/W mice and then incubated with different concentrations of an anti-mouse CD3/100bp-oligo conjugate consisting of dsDNA oligo conjugated to anti-mouse CD3 antibody. 6 hours after incubation, cells were analyzed by flow cytometry.

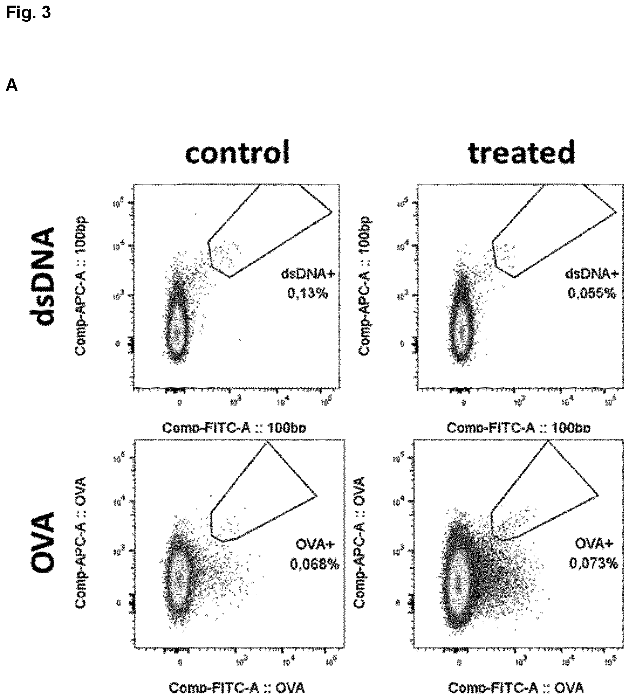

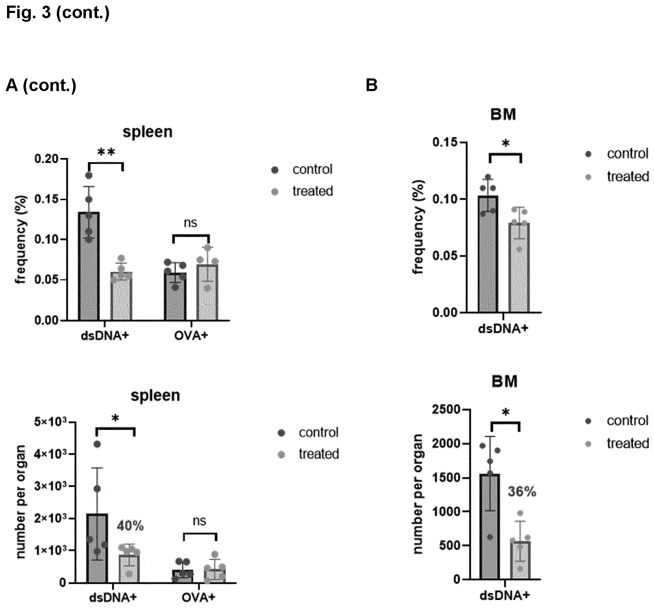

**Figure 3****:** Selective depletion of dsDNA-specific B cells *in vivo.* Lupus-prone NZB/W mice immunized with ovalbumin (OVA) were treated with a single dose of the anti-mouse CD3/100bp-oligo conjugate (200 µg/mouse), at day 11 after immunization. (A) Splenic anti-dsDNA-specific and anti-OVA-specific B cells were analyzed by flow cytometry 24 hours after treatment. (B) anti-dsDNA-specific B cells in BM were analyzed by flow cytometry at the same time. The numbers in red represented the percentage of remaining proportions in the treated group compared to the control group.

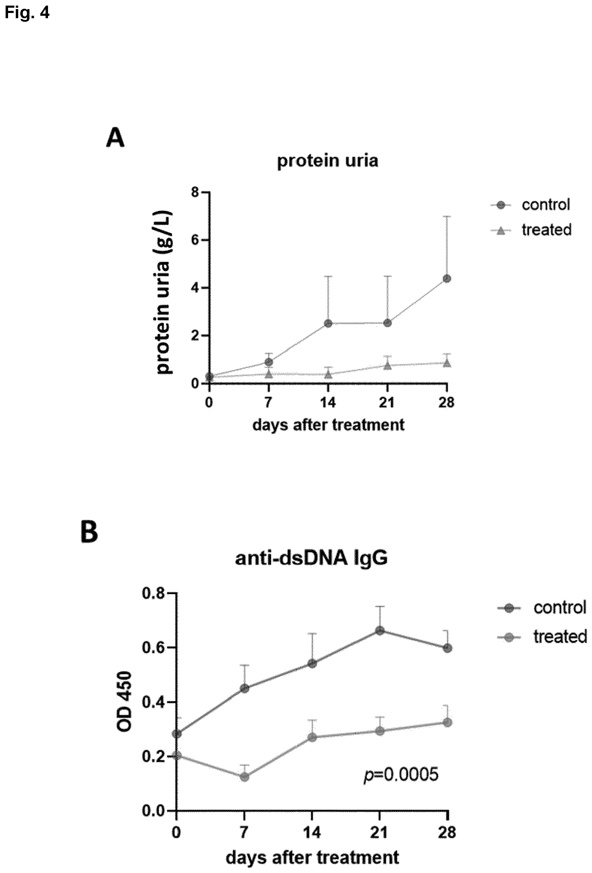

**Figure 4****:** Long-term clinical effect of selective depletion of anti-dsDNA-specific B cells *in vivo.* Lupus-prone NZB/W mice were treated with the anti-mouse CD3/100bp-oligo conjugate (200 µg/mouse) at days 0 and 14. A) Proteinuria were monitored by Albustix strips. B) anti-dsDNA IgG levels in sera were analyzed by Elisa.

### EXAMPLES

The following examples further describe the invention. These are not intended to limit the scope of the invention but represent preferred embodiments of aspects of the invention provided for a greater illustration of the invention described herein.

In order to demonstrate the functionality and beneficial properties of the bispecific conjugate described herein, the following examples are to be considered:
- Binding affinity of anti-dsDNA antibodies with distinct lengths of nucleic acid oligomers
- Selective depletion of anti-dsDNA-specific B cells ex *vivo.*
- Selective depletion of anti-dsDNA-specific B cells *in vivo.*
- Long-term clinical effect of selective depletion of dsDNA-specific B cells *in vivo.*

### Example 1: Binding affinity of anti-dsDNA antibodies with distinct lengths of nucleic acid oligomers.

A 96-well ELISA plate was first coated with methyl-BSA (Sigma Aldrich) and subsequently with calf thymus DNA (Sigma Aldrich), then blocked with 3% BSA/PBS. The sera sample from anti-dsDNA-positive SLE patients were incubated with the different lengths of nucleic acid oligomers ('non' control, 9bp, 21bp, 30bp, 40bp, 61bp, 80bp, 100bp, and 140bp, Biomers) in the final concentration of 200nM at 37°C for 2 hours, and then added to the prepared ELISA plate. After washing, the plate was incubated with HRP-labeled goat anti-human IgG (SouthernBiotech). TMB substrate (Thermo Scientific) was added for development. Extinction was measured at 450 nm by SpectraMax Plus384 (Molecular Devices) after the reaction was stopped. The figure was generated using GraphPad Prism 9 (GraphPad, La Jolla, California, USA).

### Results for the binding affinity of anti-dsDNA antibodies with distinct lengths of nucleic acid oligomers:

The interaction between nucleic acid oligomers and anti-dsDNA antibodies depends on the length of the nucleic acid oligomers. Notably, even relatively short nucleic acid oligomers of 9 bp demonstrate effective binding to anti-dsDNA antibodies. However, when the length of the nucleic acid oligomers ranges from 61 bp up to 140 bp, the binding interaction becomes markedly and significantly specific.

### Example 2: Selective depletion of anti-dsDNA-specific B cells ex vivo.

Mouse splenocytes were isolated from 24 weeks-old lupus-prone NZB/W mice and suspended in RPMI 1640 medium supplemented with 10% fetal calf serum (Invitrogen), penicillin, streptomycin, and glutamine (complete medium). Cells were split into 96-well plates (2×10⁶cell/well) and mixed with different concentrations of anti-mouse CD3/100bp-oligo conjugate (final concentration: 25, 5, 1, 0.2, 0.04, 0.008, 0.0016 and 0 µg/ml) consisting of 100 bp-oligo conjugated to anti-mouse CD3 antibody.

The conjugate, consisting of an anti-mouse CD3 antibody (clone: KT3, DRFZ) and 100 bp (Biomers), was manufactured in-house (Deutsches Rheuma-Forschungszentrum, Berlin, DRFZ). For conjugation, the anti-mouse CD3 antibody was activated by succinimidyl 4-(N-maleimidomethyl) cyclohexane-1-carboxylate (ThermoFisher Scientific) and then incubated overnight with 100 bp-Thiol-C6 (Biomers), and the conjugate was then purified using a Superdex column (GE Healthcare Life Sciences).
100 bp oligo sequence:

CTGCCAAGCTGAACATAACCGACTGGGTAGCCCCGATTGATGGATAAAACTAAAGCGAGAC
TTACTGAAGATACTAAAGGAAAGCGTACTGTGACTCTCA (SEQ ID NO 1)

After incubation at 37°C for 6 hours in a 5% CO₂-containing incubator, cells were harvested and stained with Zombie Violet (Biolegend), anti-mouse CD19-APC/CY7 (Biolegend), anti-mouse CD3-PerCP (Biolegend), 100 bp-6Fam (Biomers) and 100 bp-Cy5 (Biomers), and analyzed by FACSCanto II cytometer (BD Biosciences). The data were analyzed with FlowJo software (Tree Star Inc.). After excluding dead cells by Zombie Violet, the 100 bp (dsDNA)-specific B cell frequencies in CD19+ B cells were determined by 100 bp-6Fam and 100 bp-Cy5 double positive population. The remaining cell percentages were calculated by comparing with the mean results from 0 µg/ml. The figure was generated using GraphPad Prism 9 (GraphPad, La Jolla, California, USA).

### Results for the selective depletion of anti-dsDNA-specific 8 cells ex vivo:

The outcomes demonstrate a selective depletion of dsDNA-specific B cells that is dosedependent. Notably, a minor reduction in dsDNA-specific B cells is detectable at a concentration as low as 0.0016 µg/ml. This depletion becomes more pronounced and apparent at a concentration of 0.008 µg/ml and reaches its plateau at 0.2 µg/ml.

### Example 3: Selective depletion of anti-dsDNA-specific B cells in vivo.

Eleven days after ovalbumin (OVA) immunization, one group of 24-26 weeks old NZB/W mice were treated intraperitoneally with anti-mouse CD3/100bp-oligo conjugate (200µg/mouse, n=5).

24 hours after treatment, the cells from the spleen and bone marrow (BM) of both groups were isolated and stained with Zombie Violet (Biolegend), anti-mouse CD19-APC/CY7 (Biolegend), anti-mouse CD3-PerCP (Biolegend), 100 bp-6Fam (Biomers), 100 bp-Cy5 (Biomers), OVA-FITC (DRFZ) and OVA-Cy5 (DRFZ), and analyzed by FACSCanto II cytometer (BD Biosciences). The data were analyzed with FlowJo software (Tree Star Inc.).

After excluding dead cells by Zombie Violet, the 100 bp (dsDNA)-specific and OVA-specific B cell frequencies in CD19+ B cells were determined by 100 bp-6Fam and 100 bp-Cy5 double-positive population and OVA-FITC and OVA-Cy5 double-positive population. Absolute cell numbers were calculated based on population frequencies and total cell numbers per organ. Statistical analyses and figures were generated using GraphPad Prism 9 (GraphPad, La Jolla, California, USA). The Mann-Whitney U-test was used for statistical analysis (*p<0.05, **p<0.01).

### Results for selective depletion of dsDNA-specific B cells in vivo:

Figure 3A: Using flow cytometry, splenic B cells specific for dsDNA and ovalbumin (OVA) 24 hours post-treatment were examined. The results presented in this example indicate a substantial reduction in both the frequency and absolute count of dsDNA-specific B cells following treatment, while OVA-specific B cells remained unaffected.

Figure 3B: Employing flow cytometry, dsDNA-specific B cells in the bone marrow (BM) were assessed at the same time point. The analysis revealed a significant reduction in the frequency and absolute number of dsDNA-specific B cells. The values highlighted in red indicate the remaining proportions in the treated group relative to the control group.

### Example 4: Long-term clinical effect of selective depletion of dsDNA-specific B cells in vivo.

20 week-old NZB/W mice were treated intraperitoneally with anti-mouse CD3/100bp-oligo conjugate (200µg/mouse, n=10) at days 0 and 14. Proteinuria was monitored and semiquantitatively analyzed using Albustix (Siemens) as a biomarker of nephritis every 7 days (A). Sera were collected every 7 days, and anti-dsDNA IgG levels were analyzed by ELISA (B). 96-well ELISA plates were first coated with methyl-BSA (Sigma Aldrich) and subsequently with calf thymus DNA (Sigma Aldrich), then blocked with 3% BSA/PBS. The sera samples from both groups were added to the plates and incubated at 37°C for 2 hours. After washing, the plates were incubated with HRP-labeled goat anti-mouse IgG (SouthernBiotech). TMB substrate (Thermo Scientific) was added for development. Extinction was measured at 450 nm by SpectraMax Plus384 (Molecular Devices) after the reaction was stopped. Statistical analyses and figures were generated using GraphPad Prism 9 (GraphPad, La Jolla, California, USA). The Two-Way ANOVA test was used for statistical analysis.

### Results for long-term clinical effect of selective depletion of dsDNA-specific B cells in vivo:

Both proteinuria and anti-dsDNA IgG levels were lower in the treated group. These results suggest that the treatment involving the selective depletion of dsDNA-specific B cells either prevents or delays the onset of lupus nephritis.

### REFERENCES

1. Ellebrecht CT, Bhoj VG, Nace A, Choi EJ, Mao X, Cho MJ, Di Zenzo G, Lanzavecchia A, Seykora JT, Cotsarelis G, Milone MC, Payne AS. Reengineering chimeric antigen receptor T cells for targeted therapy of autoimmune disease. Science. 2016 Jul 8;353(6295):179-84. doi: 10.1126/science.aaf6756. Epub 2016 Jun 30. PMID: 27365313; PMCID: PMC5343513.
2. Zocher M, Baeuerle PA. A bispecific single-chain antibody fusion protein for targeted depletion of autoreactive B cells via unstimulated human T lymphocytes. Mol Immunol. 2004 Jul;41(5):511-8. doi: 10.1016/j.molimm.2004.04.002. PMID: 15183929.
3. Schett G, Mackensen A, Mougiakakos D. CAR T-cell therapy in autoimmune diseases. Lancet. 2023 Sep 22:S0140-6736(23)01126-1. doi: 10.1016/S0140-6736(23)01126-1. Epub ahead of print. PMID: 37748491.
4. Chan O, Shlomchik MJ. A new role for B cells in systemic autoimmunity: B cells promote spontaneous T cell activation in MRL-Ipr/lpr mice. J Immunol. 1998 Jan 1;160(1):51-9. PMID: 9551955.
5. Merrell KT, Benschop RJ, Gauld SB, Aviszus K, Decote-Ricardo D, Wysocki LJ, Cambier JC. Identification of anergic B cells within a wild-type repertoire. Immunity. 2006 Dec;25(6):953-62. doi: 10.1016/j.immuni.2006.10.017. PMID: 17174121.
6. Sfikakis PP, Boletis JN, Tsokos GC. Rituximab anti-B-cell therapy in systemic lupus erythematosus: pointing to the future. Curr Opin Rheumatol. 2005 Sep;17(5):550-7. doi: 10.1097/01.bor.0000172798.26249.fc. PMID: 16093832.
7. Mustelin T, Lood C, Giltiay NV. Sources of Pathogenic Nucleic Acids in Systemic Lupus Erythematosus. Front Immunol. 2019 May 8;10:1028. doi: 10.3389/fimmu.2019.01028. PMID: 31139185; PMCID: PMC6519310.
8. Kuhn KA, Kulik L, Tomooka B, Braschler KJ, Arend WP, Robinson WH, Holers VM. Antibodies against citrullinated proteins enhance tissue injury in experimental autoimmune arthritis. J Clin Invest. 2006 Apr;116(4):961-73. doi: 10.1172/JCI25422. PMID: 16585962; PMCID: PMC1421345.
9. Marasandra Ramesh H, Gude SS, Venugopal S, Peddi NC, Gude SS, Vuppalapati S. The Role of Myositis-Specific Autoantibodies in the Dermatomyositis Spectrum. Cureus. 2022 Mar 8;14(3):e22978. doi: 10.7759/cureus.22978. PMID: 35415038; PMCID: PMC8990210.
10. Tong L, Koh V, Thong BY. Review of autoantigens in Sjögren's syndrome: an update. J Inflamm Res. 2017 Aug 7;10:97-105. doi: 10.2147/JIR.S137024. PMID: 28848359; PMCID: PMC5557906.
11. Liem SIE, Neppelenbroek S, Fehres CM, Wortel C, Toes REM, Huizinga TWJ, Scherer HU, de Vries-Bouwstra JK. Autoreactive B cell responses targeting nuclear antigens in systemic sclerosis: Implications for disease pathogenesis. Semin Arthritis Rheum. 2023 Feb;58:152136. doi: 10.1016/j.semarthrit.2022.152136. Epub 2022 Nov 14. PMID: 36403538.

## Claims

1. A bispecific conjugate, comprising:
a. a binding agent that binds a T cell, and
b. an antigen that binds an autoreactive B cell, wherein the antigen is a nucleic acid oligomer.

2. The bispecific conjugate according to claim 1, wherein the binding agents binds a component of the T cell receptor (TCR).

3. The bispecific conjugate according to any one of the preceding claims, wherein the binding agent binds and activates a cytotoxic T cell.

4. The bispecific conjugate according to any one of the preceding claims, wherein the binding agent is an antibody or an antigen binding fragment thereof.

5. The bispecific conjugate according to any one of the preceding claims, wherein the binding agent is an anti-CD3 antibody or an antigen binding fragment thereof.

6. The bispecific conjugate according to any one of the preceding claims, wherein the binding agent is an anti-CD4 or anti-CD8 antibody or an antigen binding fragment thereof.

7. The bispecific conjugate according to any one of the preceding claims, wherein the antigen is a ssDNA oligomer or a dsDNA oligomer, preferably a dsDNA oligomer.

8. The bispecific conjugate according to any one of the preceding claims, wherein the nucleic acid oligomer has a length of 20 to 300 nucleotides or base pairs, preferably 60 to 200 nucleotides or base pairs, more preferably 80 to 120 nucleotides or base pairs.

9. The bispecific conjugate according to any one of the preceding claims, wherein the nucleic acid oligomer antigen is associated with an autoimmune disease, wherein the autoimmune disease is preferably a systemic autoimmune disease comprising anti-nucleic acid autoantibodies, more preferably systemic lupus erythematosus (SLE).

10. The bispecific conjugate according to any one of the preceding claims, wherein the binding agent and the antigen are associated by a covalent linker.

11. The bispecific conjugate according to any one of the preceding claims, wherein the nucleic acid oligomer is a dsDNA oligomer with a length of 80 to 120 base pairs, and wherein the binding agent is an antibody or antibody fragment that binds and activates a cytotoxic T cell, and wherein the binding agent and the antigen are associated by a covalent linker.

12. The bispecific conjugate according to any one of the preceding claims for use in the treatment and/or prevention of an autoimmune disease, preferably a systemic autoimmune disease, more preferably a systemic autoimmune disease comprising anti-nucleic acid autoantibodies.

13. The bispecific conjugate for use according to the preceding claim, wherein the autoimmune disease is systemic lupus erythematosus (SLE).

14. A pharmaceutical composition for use in the treatment and/or prevention of an autoimmune disease comprising the bispecific conjugate according to any one of claims 1 to 10 and a pharmaceutically acceptable carrier.

15. An *in vitro* method for killing an autoreactive B cell, the method comprising contacting:
- a mixture of an autoreactive B cell and a cytotoxic T cell with the bispecific conjugate according to claim 1 or with a composition comprising said conjugate,
- wherein the antigen of said bispecific conjugate binds the autoreactive B cell and the binding agent of said bispecific conjugate binds and activates the cytotoxic T cell.
